# FASCICULE DE BREVET EUROPEEN

(11) **EP 2 011 527 B1**
(45) Date de publication et mention de la délivrance du brevet: **24.11.2010**
(21) Numéro de dépôt: 08352013.0
(22) Date de dépôt: 13.06.2008
(51) Int. Cl.: A61L 31/14, A61F 2/30, C08G 63/12, C08G 63/20, B29C 67/24

(54) **Procédé de fabrication d'un article biodégradable massif à usage médical**
Herstellungsverfahren eines biologisch abbaubaren massiven Gegenstandes zur medizinischen Verwendung
Method of manufacturing a biodegradable massif article for medical use

(30) Priorité: 02.07.2007 FR 0704752; 02.07.2007 US 772336
(43) Date de publication de la demande: 07.01.2009
(73) Titulaire: Teknimed SAS, 65500 Vic en Bigorre (FR)
(72) Inventeur: Leonard, Alain, 65500 Caixon (FR); Sender, Cyril, 31400 Toulouse (FR)
(74) Mandataire: Morelle, Guy Georges Alain

(56) Documents cités:
- WO-A-03/064496
- WO-A-03/089492
- FR-A- 2 763 500
- NAGATA, M. ET AL.: "Synthesis and enzymatic degradation of regular network aliphatic polyesters" REACTIVE & FUNCTIONAL POLYMERS, vol. 30, 1996, pages 165-171, XP002468752

## Description

La présente invention se rapporte au domaine des dispositifs médicaux utilisés en chirurgie osseuse, et en particulier aux systèmes d'obturation du canal médullaire utilisés lors du scellement d'une prothèse. Elle a pour objet un procédé de préparation d'un article biodégradable à usage médical, tel qu'un obturateur diaphysaire, dans lequel du poly(glycerol sebacate) est formé dans des moules élastiques de surface totalement lisse.

Les prothèses totales (de hanche, genou...) permettent de remplacer une articulation endommagée et ainsi de soulager la douleur et d'améliorer la mobilité du patient. Les principales indications de pose de ce type d'implant sont les dégénérescences articulaires et certaines fractures.

Lors d'une arthroplastie totale de hanche cimentée, une prothèse est introduite dans le canal médullaire et y est fixée à l'aide d'un ciment acrylique, généralement à base de PMMA (polyméthylméthacrylate de méthyle). La réaction de polymérisation du monomère se produit en quelques minutes. La mise en place d'une prothèse totale de hanche cimentée nécessite une bonne préparation des surfaces osseuses et un remplissage homogène et total du canal proximal avec le ciment acrylique. L'introduction de la prothèse dans le canal médullaire rempli de ciment induit une mise sous pression de ce dernier qui a tendance à s'échapper de la zone implantée par l'orifice proximal ou en venant au contact de la moelle osseuse. Ces vides sont responsables de phénomènes de concentrations de contraintes et de points de rupture. Pour éviter ces phénomènes, l'utilisation d'un obturateur diaphysaire placé dans le canal médullaire au fond de la zone implantée est nécessaire. La fonction première de l'obturateur est d'éviter la formation de ces zones vides de ciment autour de la prothèse en limitant la progression de ciment dans la partie distale. Il permet non seulement d'éviter la diffusion du PMMA et de son monomère dans la moelle osseuse mais aussi d'augmenter la pression intramédullaire à l'impaction de la prothèse et donc de favoriser une bonne stabilité de la prothèse par interpénétration du ciment dans les tissus osseux environnants.

L'intérêt de l'occlusion du fût fémoral lors d'une arthroplastie totale de hanche cimentée est aujourd'hui parfaitement établi. L'obturateur doit être stérile, biocompatible et suffisamment souple pour s'adapter aux irrégularités de forme du canal diaphysaire. L'obturation a été réalisée avec différents types de bouchons décrits dans la technique:
- bouchon en ciment acrylique,
- bouchon en polyéthylène (PE), silicone, polyéthylène téréphtalate (PET), ou encore en polyétheréthercétone (PEEK),
- bouchon d'os spongieux, prélevé à partir de la pièce d'ostéotomie ou creusé dans la partie supérieure du canal médullaire,
- bouchon en matière biorésorbable à base de gélatine, d'acide polylactique ou de copolymère PEGT/PBT (Polyactive^{™}).

Selon leur nature, ces obturateurs présentent des inconvénients. Certains sont trop rigides et ne permettent pas d'épouser suffisamment la forme du canal fémoral pour éviter les fuites de ciment. Cette importante rigidité confère également à ces systèmes une tendance à la migration distale lors de l'impaction de la prothèse. Ils sont majoritairement non biodégradables ce qui gêne l'intervention chirurgicale lors d'une éventuelle reprise ultérieure. En effet, cette extraction doit être réalisée sans générer de débris responsables de phénomène d'ostéolyse bien connus, et allonge la durée de l'intervention.

Les obturateurs biorésorbables en acide polylactique peuvent provoquer des réponses inflammatoires chez certains patients et l'emploi d'os spongieux est proscrit car on observe fréquemment la formation d'un pont osseux difficile à retirer.

Les bouchons à base de gélatine ne présentent pas ce type d'inconvénients et sont actuellement largement utilisés. Néanmoins, étant majoritairement constitués de gélatine d'origine porcine, ils peuvent être considérés comme véhicules potentiels d'entités pathologiques (prions...) et revêtent pour certains patients un caractère allergène. Les exigences de sécurité sanitaire imposent désormais de proposer des dispositifs médicaux exempts de toute incertitude quant à leur innocuité, conformément au principe de précaution. Enfin, leur emploi sur des patients de religion juive ou musulmane peut être mal apprécié. La limitation ou l'interdiction de ce type de produit doit être prévenue par la mise au point d'un nouvel obturateur.

L'obturateur obtenu selon le procédé objet de l'invention est doté de toutes les propriétés physico-chimiques et mécaniques nécessaires à sa fonction, il est totalement exempt de toute substance susceptible de présenter un risque pour la santé des patients. Le procédé selon la présente invention permet ainsi d'obtenir un polymère de substitution à la formulation à base de gélatine porcine actuellement utilisée. Après la réalisation d'essais préliminaires en laboratoire sur divers systèmes, il a été découvert que le polymère appelé PGS pour poly(glycerol sebacate) répondait à l'ensemble des critères du cahier des charges défini plus haut, à condition que sa fabrication soit réalisée dans des conditions bien définies permettant d'obtenir des objets massifs compacts et homogènes, dépourvus de défauts de structure. Ce nouveau matériau est entièrement synthétique, biorésorbable et présente des propriétés élastiques proches du produit actuel.

Le PGS ainsi fabriqué est obtenu par polycondensation directe du glycérol et de l'acide sébacique. Il est connu et utilisé dans le domaine de l'ingénierie tissulaire comme support de culture. Toutefois, son application est limitée sous forme de films minces nervurés pour guider la formation des tissus. Il est également utilisé sous forme de fil, de particules, de tube, de fibre, ou de trames tissées. Seuls des objets de très faible épaisseur sont obtenus par le procédé de polymérisation décrit jusqu'à présent dans l'état de la technique (WO 03/064496). Ce procédé consiste à faire condenser un mélange de glycérol et d'acide sébacique à 120°C suivant un cycle de pression en deux étapes dans des moules rigides en polytetrafluoroéthylène (PTFE) où un agent de démoulage hydrosoluble est préconisé. Or, pour des objets massifs de dimension centimétrique et de géométrie complexe, des moules composés de plusieurs parties doivent classiquement être utilisés. Néanmoins, lorsque le mélange atteint le point de gel et alors qu'il est encore visqueux et sans tenue mécanique, la présence de bulles favorisées par les conditions de température et de pression au niveau du ou des plans de joint à la jonction des différentes parties du moule déforment l'objet moulé aboutissant à une masse hétérogène pourvue de défauts volumiques et surfaciques qui fragilisent l'objet et réduisent ses propriétés de résistance mécanique. L'expression « point de gel » désigne le moment où la masse molaire moyenne du polymère ainsi que sa viscosité augmentent brutalement vers l'infini.

Il n'a donc pas été possible à ce jour de fabriquer des obturateurs diaphysaires biocompatibles, biorésorbables, et totalement synthétique qui soient massifs et homogènes, exempts de défauts de structure, et dont les propriétés mécaniques soient voisines des produits actuels à base de gélatine. La présente invention résout ces problèmes grâce à un procédé de fabrication d'un polymère à base de PGS, dont les conditions opératoires permettent d'obtenir un objet massif de forme aussi complexe soit-elle, compact et homogène. Il a été en particulier mis en évidence qu'il était primordial d'empêcher la formation de bulles lors du coulage du mélange de monomères dans les moules et que le choix des moules revêtait une importance capitale.

Le terme compact désigne le caractère d'un objet de structure tridimensionnelle massive pleine par opposition à une structure poreuse ou lacunaire, ou sous forme de film ou de fil. L'homogénéité indique que le caractère compact est uniforme dans toute la masse de l'objet en question. Il découle de ces deux caractères une structure dépourvue de défauts de structure interne ou de surface, n'offrant pas de zone de moindre résistance ou de moindre élasticité susceptible de favoriser la rupture par déchirement.

Plus précisément, est objet de la présente invention un procédé de préparation d'un article biodégradable massif à usage médical comprenant les étapes consistant à :
a) préparer un mélange comprenant du glycérol et de l'acide sébacique, sans l'utilisation d'un quelconque solvant,
b) verser ledit mélange dans un moule constitué d'une seule pièce (quelle que soit la complexité dimensionnelle de l'objet à mouler), en matériau élastique,
c) placer le moule contenant ledit mélange sur un support en matériau thermiquement conducteur,
d) placer le support soutenant le moule contenant ledit mélange dans une enceinte à vide à plateaux chauffants à une température comprise entre 80 et 150 °C, sous une pression comprise entre 5 et 500 mbar, jusqu'à obtention d'un polymère de consistance adaptée à sa fonction (semblable à celle de l'obturateur diaphysaire CEMSTOP^{®} à base de gélatine),
e) retirer le support de l'enceinte et laisser refroidir à température ambiante, et
f) procéder au démoulage de l'article ainsi obtenu, par ouverture (éventuellement déchirement) et décollement de la paroi élastique du moule en contact avec l'objet moulé.

La polycondensation directe du glycérol et de l'acide sébacique, c'est-à-dire respectivement d'un triol et d'un diacide carboxylique linéaire, aboutit à la formation d'un copolymère thermodurcissable. A température ambiante, l'acide sébacique se présente sous forme pulvérulente (sa température de fusion est de 130°C). Le glycérol est quant à lui un liquide visqueux. Le mélange de deux monomères peut se faire en amenant l'acide sébacique à l'état liquide et en le mélangeant avec le glycérol. Ce mode opératoire présente l'avantage de ne faire intervenir aucun solvant dont les traces pourraient conduire au relargage de substances nocives ou toxiques dans l'organisme après implantation de l'obturateur. Le rapport molaire glycérol/acide sébacique est inférieur à 1. Selon une caractéristique préférée de l'invention, le mélange du glycérol et de l'acide sébacique est obtenu par liquéfaction du second dans le premier à 150°C. Cette étape peut être réalisée dans une atmosphère inerte pour éviter toute oxydation des monomères et prévenir le jaunissement du mélange.

Un avantage de la présente invention réside dans le fait que l'ensemble du procédé peut être réalisé sans solvant.

De manière préférée, les étapes a), d) et e) du procédé selon la présente invention peuvent être réalisées sous atmosphère inerte.

Le mélange liquide de monomères est versé dans un moule (en pratique dans les conditions industrielles dans une série de moules) qui présente l'originalité d'être réalisé dans un matériau élastique et d'un seul tenant. L'avantage essentiel par rapport aux moules traditionnels rigides formés de deux coques ou plus, est qu'il est dépourvu de plan de joint, qui si fin soit-il, créée une discontinuité à la jonction des deux coques. Cette discontinuité s'est avérée être à l'origine de la formation de bulles ne permettant pas d'obtenir un objet compact et homogène lors de la polymérisation sous pression réduite. Le plan de joint visible sur l'objet moulé constitue une zone de moindre résistance à l'origine d'une fragilisation de l'objet sous fortes contraintes mécaniques. L'emploi d'un moule de surface interne parfaitement lisse et sans plan de joint résout ce problème. L'élasticité des moules rend possible l'élaboration d'objets de géométrie complexe et facilite leur démoulage sans les endommager. Pour ce type d'application, il doit de plus être de grade à usage médical ou alimentaire.

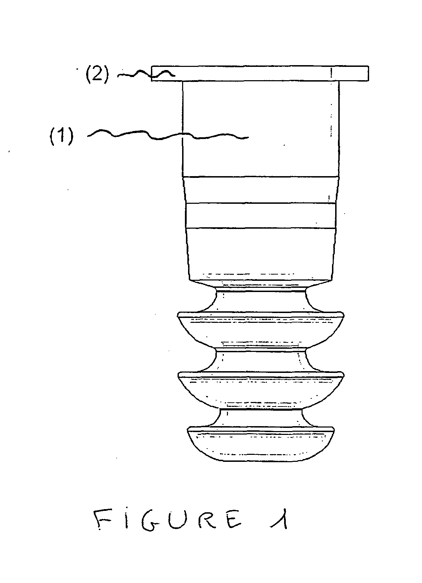

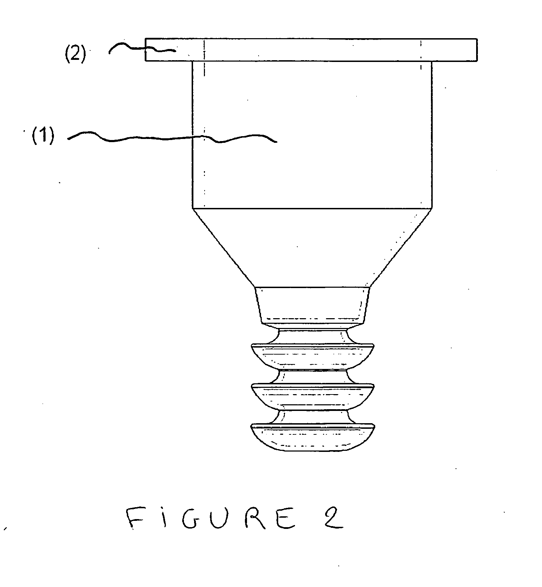

Les figures 1 et 2 représentent deux moules en silicone permettant d'obtenir des obturateurs de diamètre 8 et 18 millimètres selon le procédé objet de la présente invention.

Selon une caractéristique préférée de l'invention, le moule comprend un réservoir (1) avec un rebord supérieur (2) apte à soutenir ledit moule en appui sur son support. L'épaisseur de la paroi est choisie en fonction de la résistance au déchirement du matériau qui constitue le moule.

Selon une caractéristique avantageuse du procédé de la présente invention, le moule inséré dans son support de dimension légèrement supérieure (quelques millimètres au maximum) au plus grand diamètre extérieur de l'empreinte permet d'obtenir des articles polymérisés de façon très homogène. On note un degré de polymérisation uniforme sur toute la hauteur des articles, ce qui n'est pas le cas lorsqu'on utilise des empreintes en aluminium ou en polytetrafluoroéthylène. Ce phénomène est attribué au faible espacement entre le moule et son support favorisant des échanges thermiques radiaux donc une répartition homogène de la chaleur transmise au mélange réactionnel.

De manière préférée, le moule utilisé dans le procédé objet de l'invention présente sa section la plus élevée au niveau de l'ouverture supérieure du réservoir (1). Le support des moules est en matériau thermiquement conducteur et de géométrie interne épousant de façon intime le contour global externe des moules.

Selon une caractéristique préférée, la polymérisation du PGS est réalisée sous pression réduite d'air ou d'un gaz inerte, pour éliminer progressivement l'eau produite par la réaction de polymérisation. Ceci permet d'accélérer la cinétique de réaction et de diminuer les temps et les coûts de production. Le niveau de pression est inférieur à 500 mbar.

La température de polymérisation doit être suffisamment élevée pour ne pas que l'acide sébacique ne solidifie dans le mélange de monomères, et ne pas être trop élevée pour ne pas favoriser la formation de bulles dans le volume du mélange. Les températures minimale et maximale du procédé selon l'invention ont été évaluées à 80 et 150°C respectivement. Selon l'invention, la température de travail peut être choisie en fonction de la cinétique de la réaction de polymérisation, celle-ci étant plus rapide à haute température.

Lorsque la polymérisation a atteint le taux d'avancement conférant à l'objet moulé la consistance souhaitée, la réaction est stoppée par retour à la température ambiante. Le produit obtenu est stable, de sorte qu'on peut le démouler immédiatement ou bien le stocker en l'état. Le démoulage doit être réalisé sans endommager l'article obtenu. On met à profit l'élasticité du moule et sa faible résistance au déchirement pour retirer celui-ci en douceur. L'homme de l'art sait apprécier ce paramètre par quelques essais simples sur le matériau choisi. L'objet polymérisé, alors élastique, peut être durci par refroidissement préalable entre -15 et -25°C dans un congélateur pour faciliter cette étape.

Le procédé qui vient d'être décrit peut être mis en oeuvre pour la fabrication de tout article massif en PGS, notamment des articles biodégradables à usage médical. Il trouve une application particulièrement adaptée dans la fabrication d'un obturateur diaphysaire.

### EXEMPLE

L'exemple ci-après a pour but d'illustrer un mode de réalisation de la présente invention sans pour autant en limiter la portée.

### Synthèse d'un article massif et compact en PGS

### 1. Préparation du mélange de monomères

Le glycérol et l'acide sébacique sont introduits dans un fondoir chauffé à 150°C et balayé en surface par un flux d'azote gazeux afin d'éviter l'oxydation des monomères au contact de l'air. L'agitation est assurée par une hélice à vitesse lente pour prévenir l'introduction de bulles dans le mélange. Le mélange est maintenu sous agitation jusqu'à dissolution complète de l'acide dans le glycérol.

### 2. Coulage du mélange

Le mélange des monomères est coulé à 150°C dans les moules placés sur leur support par l'ouverture d'une vanne basse de la cuve du fondoir. Le volume de mélange est dosé en fonction de la taille de l'objet à fabriquer. L'élasticité des moules permet une déformation aisée et une évacuation manuelle d'éventuelles bulles d'air qui auraient été piégées dans les aspérités de l'empreinte. Les moules sont en silicone de grade alimentaire ou médical, d'un seul tenant et ne présentent aucune discontinuité ni rugosité de surface interne liée à leur confection.

### 3. Polymérisation

Les moules remplis et positionnés sur leur support sont disposés dans une enceinte à vide à plateaux chauffants. Ce dispositif permet de contrôler à la fois la température, le niveau de vide de l'enceinte et le maintien d'une atmosphère inerte évitant le jaunissement du PGS. La température est fixée à 130°C et la pression à 200 mbar. L'atmosphère inerte est obtenue par purges successives de l'enceinte suivant 2 à 3 cycles vidange/remplissage à l'aide d'une pompe à vide et d'une réserve en gaz neutre reliées à l'enceinte. L'eau produite par la réaction de polycondensation des monomères est évacuée par évaporation à l'interface du milieu réactionnel avec le gaz environnant. Le temps de polymérisation est compris entre 2 et 3 jours pour un volume de PGS de 1 mL.

Le produit obtenu est un élastomère ayant des propriétés élastiques comparables à celles de la gélatine porcine actuellement employée pour la fabrication des obturateurs diaphysaires (type CEMSTOP^{®}). Les articles moulés ne présentent aucun défaut de surface, ni inclusion gazeuse susceptible de nuire à leur bonne qualité, notamment au regard de la résistance au déchirement.

## Revendications

1. Procédé de préparation d'un article biodégradable massif à usage médical **caractérisé en ce qu'**il comprend les étapes consistant à :
a) préparer un mélange comprenant du glycérol et de l'acide sébacique ;
b) verser ledit mélange dans un moule constitué d'une seule pièce en matériau élastique ;
c) placer ledit moule contenant ledit mélange sur un support ;
d) placer le support dans une enceinte à température et niveau de vide contrôlés jusqu'à obtention d'un polymère de consistance souhaitée ;
e) retirer le support de l'enceinte et laisser refroidir jusqu'à température ambiante ;
f) procéder au démoulage de l'article ainsi obtenu.

2. Procédé selon la revendication précédente **caractérisé en ce que** le rapport molaire glycérol/acide sébacique est inférieur à 1.

3. Procédé selon la revendication 1 ou 2 **caractérisé en ce que** l'ensemble du procédé est réalisé sans solvant.

4. Procédé selon l'une quelconque des revendications précédentes **caractérisé en ce que** les étapes a), d) et e) sont réalisées sous atmosphère inerte.

5. Procédé selon l'une quelconque des revendications précédentes **caractérisé en ce que** l'étape d) est réalisée à une température comprise entre 80 et 150°C.

6. Procédé selon l'une quelconque des revendications précédentes **caractérisé en ce que** l'étape d) est réalisée à un niveau de pression compris entre 5 et 500 mbar.

7. Procédé selon l'une quelconque des revendications précédentes **caractérisé en ce que** le démoulage de l'article est réalisé par ouverture et décollement de la paroi élastique du moule en contact avec l'objet moulé.

8. Procédé selon l'une quelconque des revendications précédentes **caractérisé en ce que**, préalablement à la séparation du moule et de l'article moulé, le moule et l'article qu'il contient sont refroidis à une température comprise entre -15 et -25°C.

9. Procédé selon l'une quelconque des revendications précédentes **caractérisé en ce que** le moule comprend un rebord supérieur (2) apte à soutenir ledit moule en appui sur son support.

10. Procédé selon l'une quelconque des revendications précédentes **caractérisé en ce que** le moule présente sa section la plus élevée au niveau de l'ouverture supérieure du réservoir (1).

11. Procédé selon l'une quelconque des revendications précédentes **caractérisé en ce que** le support des moules est en matériau thermiquement conducteur et de géométrie interne épousant de façon intime le contour global externe des moules.

## Claims

1. Method of preparation of a solid biodegradable article for medical use, **characterised in that** it comprises the steps consisting of:
a) preparing a mixture comprising glycerol and sebacic acid;
b) pouring said mixture into a mould consisting of one piece in elastic material;
c) placing said mould containing said mixture on a support;
d) placing the support in an enclosure with controlled temperature and vacuum level until a polymer of required consistency is obtained;
e) removing the support from the enclosure and allowing to cool to room temperature;
f) proceeding to remove from the mould the article thus obtained.

2. Method according to the preceding claim, **characterised in that** the glycerol/sebacic acid molar ratio is less than 1.

3. Method according to claim 1 or 2, **characterised in that** the whole of the method is carried out without solvent.

4. Method according to any of the preceding claims, **characterised in that** steps a), d) and e) are carried out in an inert atmosphere.

5. Method according to any of the preceding claims, **characterised in that** step d) is carried out at a temperature between 80 and 150°C.

6. Method according to any of the preceding claims, **characterised in that** step d) is carried out at a pressure level between 5 and 500 mbar.

7. Method according to any of the preceding claims, **characterised in that** the removal of the article from the mould is effected by opening and unsticking the elastic wall of the mould in contact with the moulded object.

8. Method according to any of the preceding claims, **characterised in that**, before separating the mould and the moulded article, the mould and the article that it contains are cooled to a temperature between -15 and -25°C.

9. Method according to any of the preceding claims, **characterised in that** the mould comprises an upper rim (2) adapted to support said mould by resting on its support.

10. Method according to any of the preceding claims, **characterised in that** the mould has its highest section at the level of the upper opening of the reservoir (1).

11. Method according to any of the preceding claims, **characterised in that** the support of the moulds is in thermally-conductive material and of internal geometry espousing intimately the overall external contour of the moulds.

## Patentansprüche

1. Verfahren zum Bereitstellen eines biologisch abbaubaren massiven Gegenstands zur medizinischen Verwendung
**dadurch gekennzeichnet,**
**dass** es Stufen aufweist, bestehend aus:
a) Bereitstellen einer Mischung, die Glycerin und Sebacinsäure enthält;
b) Gießen der Mischung in eine Form, die aus einem Stück aus elastischem Material gebildet ist;
c) Anordnen der Form, die die Mischung enthält, auf einem Träger;
d) Anordnen des Trägers innerhalb einer Umhüllung bei einer Temperatur und einem Unterdruck, die bis zum Erhalt eines Polymeren der gewünschten Konsistenz kontrolliert werden;
e) Zurückziehen des Trägers aus der Umhüllung und Abkühlenlassen auf Umgebungstemperatur;
f) Aus der Form nehmen des so erhaltenen Gegenstands.

2. Verfahren nach dem vorhergehenden Anspruch,
**dadurch gekennzeichnet,**
**dass** das Molverhältnis Glycerin/Sebacinsäure unter 1 liegt.

3. Verfahren nach Anspruch 1 oder 2,
**dadurch gekennzeichnet,**
**dass** das gesamte Verfahren ohne Lösungsmittel durchgeführt wird.

4. Verfahren nach einem der vorhergehenden Ansprüche,
**dadurch gekennzeichnet,**
**dass** die Stufen a), d) und e) unter Schutzgas durchgeführt werden.

5. Verfahren nach einem der vorhergehenden Ansprüche,
**dadurch gekennzeichnet,**
**dass** die Stufe d) bei einer Temperatur zwischen 80 und 150 °C durchgeführt wird.

6. Verfahren nach einem der vorhergehenden Ansprüche,
**dadurch gekennzeichnet,**
**dass** die Stufe d) bei einer Druckhöhe zwischen 5 und 500 mbar durchgeführt wird.

7. Verfahren nach einem der vorhergehenden Ansprüche,
**dadurch gekennzeichnet,**
**dass** das aus der Form nehmen des Gegenstands durch Öffnung und Ablösung der elastischen Wandung der Form, die mit dem geformten Objekt in Berührung ist, durchgeführt wird.

8. Verfahren nach einem der vorhergehenden Ansprüche,
**dadurch gekennzeichnet,**
**dass** vor der Trennung der Form und des geformten Gegenstands die Form und der Gegenstand, den sie enthält, auf eine Temperatur zwischen -15 und -25 °C abgekühlt werden.

9. Verfahren nach einem der vorhergehenden Ansprüche,
**dadurch gekennzeichnet,**
**dass** die Form einen oberen Rand (2) aufweist, der geeignet ist, die Form in Auflage auf ihrem Träger zu halten.

10. Verfahren nach einem der vorhergehenden Ansprüche,
**dadurch gekennzeichnet,**
**dass** der höchste Abschnitt der Form auf der Höhe der oberen Öffnung des Behälters (1) liegt.

11. Verfahren nach einem der vorhergehenden Ansprüche,
**dadurch gekennzeichnet,**
**dass** der Träger der Formen aus einem Wärme leitenden Material ist und eine innere Geometrie aufweist, die sich eng an die äußere Gesamtkontur der Formen anschmiegt.
